(19) Europäisches Patentamt
European Patent Office
Office européen des brevets

(11) Numéro de publication : **0 334 402 B1**

(12) **FASCICULE DE BREVET EUROPEEN**

(45) Date de publication du fascicule du brevet :
**15.01.92 Bulletin 92/03**

(51) Int. Cl.[5] : **A61K 35/78, // (A61K35/78, 31:195)**

(21) Numéro de dépôt : **89200280.9**

(22) Date de dépôt : **07.02.89**

(54) **Composition antiasthénique, son procédé de préparation et son utilisation.**

(30) Priorité : **25.02.88 BE 8800222**

(43) Date de publication de la demande :
**27.09.89 Bulletin 89/39**

(45) Mention de la délivrance du brevet :
**15.01.92 Bulletin 92/03**

(84) Etats contractants désignés :
**AT BE CH DE ES FR GB GR IT LI LU NL SE**

(56) Documents cités :
**UNLISTED DRUGS, vol. 33, no. 8, août 1981, page 132, Chatham, New Jersey, US**

(56) Documents cités :
**UNLISTED DRUGS, vol. 25, no. 2, février 1973, page 17, Chatham, New Jersey, US DICTIONNAIRE VIDAL, 1984, pages 7,593, O.V.P. Paris, FR**

(73) Titulaire : "THERABEL RESEARCH SA/NV"
**Chaussée d'Alsemberg, 1001**
**B-1180 Bruxelles (BE)**

(72) Inventeur : **Geczy, Joseph**
**Avenue de Wolvendael, 21**
**B-1180 Bruxelles (BE)**

(74) Mandataire : **Schmitz, Yvon et al**
**Bureau Gevers S.A. rue de Livourne 7 bte 1**
**B-1050 Bruxelles (BE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 334 402 B1

## Description

La présente invention est relative à une composition antiasthénique, à son procédé de préparation et à son utilisation.

Comme on le sait, le ginseng est utilisé depuis des millénaires dans les médecines traditionnelles orientales (Chine, Inde, Corée, Japon,...). Mentionné pour la première fois en 1711 en France, il figure dans la 9ème édition de la Pharmacopée française de 1974. Comme il s'agit d'un produit composé, de composition non standardisée, souvent méconnu des praticiens occidentaux, son utilisation comme médicament n'est pas entrée dans les coutumes. Ceci explique le peu d'études cliniques effectuées sur le ginseng, malgré l'existence d'études fondamentales mettant en exergue une activité pharmacologique indéniable. Le ginseng (Panax ginseng) est en fait composé de saponides dont le panaxadiol et le panaxatriol, de ginsensosides $A_1$, $B_1$, $B_2$, C, $Rb_1$ et $R_e$, de vitamines $B_1$ et $B_2$, de phytostérol et d'essence volatile (panacène). Il a été démontré chez le rat que le ginseng a une action positive sur le métabolisme énergétique durant l'effort. L'effet se produit par une action sur le métabolisme intermédiaire des hydrates de carbone et les lipides ; durant l'effort, des taux de glycémie plus élevés et une diminution des taux d'acides gras libres ainsi que d'acides lactique et pyruvique ont été démontrés chez des animaux ayant préalablement reçu 2 mg/100 mg d'extrait de ginseng par voie intrapéritonéale. Ceci indique que le ginseng est capable d'altérer les mécanismes d'homéostase énergétique durant l'effort prolongé, probablement en augmentant la capacité biochimique du muscle strié à oxyder les acides gras libres préférentiellement au glucose pour la production d'énergie cellulaire. En utilisant les fibroblastes diploïdes humains, Shia et al. (The effects of ginseng saponins on the growth and metabolism of human diploid fibroblasts, Gerontology 28 ; 121-124 (1982)), ont démontré que dans les cellules cultivées dans un milieu contenant du ginseng, l'activité de la phosphohexose isomérase et de la lactate hydrogénase est accrue comparativement à des cellules témoins. Un des processus de vieillissement cellulaire consiste en une capacité décrue de tolérer des conditions d'anaérobiose. En augmentant l'activité de la lactate déshydrogénase, le ginseng replace les organes vieillissants dans des conditions où les mécanismes anaérobiques sont restitués, donc capables de soutenir des contraintes prolongées. Ceci influence les organes vitaux en leur permettant une utilisation d'énergie plus efficace mais sans nécessairement influencer la longévité. L'action du ginseng sur le stress se traduit par une atténuation de la réaction d'alarme du sujet due à un effet sur les surrénales, évitant à ces glandes une sécrétion abusive de glucocorticoïdes et d'adrénaline. Les fonctions sexuelles sont favorablement influencées d'une part par l'équilibre émotionnel induit, par la suppression de la sensation de fatigue, tant physique qu'intellectuelle, et d'autre part par une augmentation des taux hormonaux.

Bien que sur le système nerveux, le ginseng ne se comporte pas comme un psychoanaleptique mais plutôt comme un régulateur de la stabilité et qu'il soit généralement bien toléré, on a constaté, lorsqu'il était utilisé en grandes quantités, ce qui est généralement nécessaire pour obtenir les effets avantageux précités, notamment chez les personnes âgées, des cas fréquents de nervosité, d'insomnie, d'hypertention, de diarrhée matinale et d'éruption cutanée. Des cas d'effet comparable à l'oestradiol ont été mentionnés chez des femmes ménopausées, l'utilisation prolongée de fortes doses étant par conséquent à déconseiller en période post-ménopausale. D'autres effets secondaires ont également été signalés dans des cas isolés.

Le N-acétylasparaginate d'arginine se présente comme une combinaison de deux acides aminés. L'administration de cette substance apporte évidemment un surplus d'acides aminés au métabolisme intermédiaire. Un certain nombre de caractéristiques favorables ont en outre été attribuées à l'arginine. C'est ainsi qu'il a été démontré chez le rat que l'arginine possède un effet stimulant sur les réactions métaboliques, hormonales et immunologiques. L'administration d'arginine influence en outre positivement la guérison de plaies et la fonction immunologique post-traumatique. Un des effets sur le métabolisme intermédiaire consiste à activer des voies de métabolisation alternatives pour l'excrétion de l'azote résiduel. Dans le domaine de la fonction reproductrice, l' arginine s'est montrée un adjuvant fort apprécié dans le traitement des oligospermies et des oligoasthénospermies. Bien que le N-acétylasparaginate d'arginine soit utilisé actuellement comme antiasthénique et pour l'apport d'acides aminés, il présente l'inconvénient majeur, comme dans le cas du ginseng, de devoir être utilisé en grosses quantités et pendant des périodes de temps prolongées notamment chez les personnes âgées, avant d'agir comme stimulant métabolique et de la spermatogénèse.

Il apparaît donc que, pris isolément, le ginseng et le N-acétylasparaginate d'arginine ne présentent qu'un intérêt thérapeutique mitigé, principalement chez les personnes âgées.

Hors, on a découvert d'une façon surprenante et imprévisible, suivant la présente invention, que l'activité antiasthénique du ginseng ou du N-acétylaparaginate d'arginine est très notablement augmentée, si l'on associe ces deux substances, et qu'en outre l' utilisation concomitante de ces deux substances ne peut être que bénéfique dans toutes les formes d'asthénie, c'est-à-dire physique, mentale ou sexuelle, le ginseng opérant plutôt comme régulateur du système nerveux central et le N-acétylasparaginate d'arginine comme stimulant métabolique et de la spermatogénèse.

2

La composition antiasthénique, suivant l'invention, comprend, à cet effet, comme ingrédient actif, un mélange de ginseng (Panax ginseng) et de N-acétylasparaginate d'arginine, en association avec un excipient pharmacologiquement acceptable.

Suivant une forme avantageuse de l'invention, le ginseng est sous forme de poudre, auquel cas le rapport en poids de la poudre de ginseng par rapport au N-acétylasparaginate d'arginine est de l'ordre de 1/1.

Suivant une autre forme de réalisation de l'invention, le ginseng est sous forme d'extrait, auquel cas le rapport en poids de l'extrait de ginseng par rapport au N-acétylasparaginate d'arginine est de l'ordre de 1/5.

L'invention se rapporte également à la préparation de cette composition antiasthénique ainsi qu'à son utilisation.

Comme on vient de le mentionner précédemment, la composition antiasthénique de la présente invention est constituée d'un mélange de ginseng et de N-acétylasparaginate d'arginine, ainsi que d'un excipient pharmacologiquement acceptable, tel que, par exemple, une ou plusieurs substances choisies dans le groupe comprenant la polyvinylpyrrolidone, la cellulose microcristalline, le stéarate de magnésium, le bicarbonate sodique, l'acide citrique anhydre, l'$\alpha$-lactose anhydre, le benzoate sodique et le saccharinate sodique.

La composition antiasthénique de l'invention se présente avantageusement sous la forme de dose unitaire ou posologique contenant, comme ingrédients actifs essentiels, du ginseng et du N-acétylasparaginate d'arginine et, comme excipient, un mélange de polyvinylpyrrolidone, de cellulose microcristalline et de stéarate de magnésium ou encore un mélange de bicarbonate sodique, d'acide citrique anhydre, d'$\alpha$-lactose anhydre, de benzoate sodique et de saccharinate sodique. Comme on l'a déjà précisé précédemment, le ginseng est de préférence sous la forme de poudre mais il peut également être sous la forme d'extrait, l'extraction du ginseng rendant toutefois celui-ci moins avantageux du point de vue économique. L'arginine renfermant plusieurs centres asymétriques peut donc exister sous les formes isomères optiquement actives. Il est bien entendu que l'invention couvre les deux formes épimères, telles que les formes lévogyre et dextrogyre, ainsi que leur mélange D, L.

Les expressions "dose unitaire" et "dose posologique" telles qu'elles sont utilisées dans le présent mémoire, désignent une dose physiquement déterminée contenant une quantité déterminée de constituants actifs, cette quantité étant telle qu'une ou plusieurs doses sont nécessaires pour obtenir un effet avantageux.

D'une façon générale, la composition antiasthénique suivant l'invention se présente sous une forme posologique unitaire, telle que comprimé ou gélule, comprimé effervescent, solution buvable, dont la dose en substances actives a été préétablie de sorte que l'administration de la dose journalière de ces substances pourra se faire d'une façon très simple.

Avantageusement, cette dose est généralement de l'ordre de 2 g par jour.

Ladite composition est généralement administrée à raison de quatre doses journalières, c'est-à-dire qu'un comprimé ou une gélule contiendra environ 250 mg de ginseng et environ 250 mg de N-acétylasparaginate d'arginine. Lorsque la composition est sous la forme de solution buvable celle-ci sera généralement administrée à raison de deux doses journalières. Comme excipients, on pourrait évidemment faire usage d'autres excipients pharmacologiquement acceptables généralement connus que ceux susmentionnés, tels que le vin de quinquina, lorsque le ginseng est sous forme d'extrait et que l'on désire obtenir une solution buvable.

L'avantage des compositions ou formes posologiques unitaires de l'invention, telles que comprimés, gélules, solutions, du point de vue médical consiste donc dans la possibilité de réduire fortement les doses de produit actif au bénéfice de la santé du patient.

On donne ci-après des exemples non limitatifs de formulations galéniques pouvant être utilisées suivant l'invention à titre de composition antiasthénique destinée à l'administration par voie orale et sous forme de solution buvable.

**Exemple 1**

Comprimés

| | | |
|---|---|---|
| N-acétylasparaginate de L-arginine | 250 | mg |
| Poudre de ginseng | 250 | mg |
| Kollidon® 30 (polyvinylpyrrolidone) | 25 | mg |
| Emcocel® (cellulose microcristalline) | 25 | mg |
| Stéarate de magnésium | 2,5 | mg |

Le N-acétylasparaginate de L-arginine et la poudre de ginseng sont tamisés sur un tamis de 500 µm, le stéarate de magnésium sur un tamis de 150 µm et l'Emcocel® est employé tel quel. Les deux principes actifs (N-acétylasparaginate de L-arginine et poudre de ginseng) sont introduits dans un mélangeur planétaire et mélangés pendant 10 minutes à 70 tours par minute. On granule ensuite le mélange à l'aide d'une solution méthanolique de Kollidon® 30, la granulation étant effectuée dans le même mélangeur planétaire. On fait ensuite passer le granulé sur un tamis oscillant (750 µm) et on le sèche à 30°C. Au granulé séché, on ajoute l'Emcocel®, on mélange pendant 5 minutes à 70 tours par minute, on ajoute enfin le stéarate de magnésium et on mélange pendant 2 minutes à 50 tours par minute. Le mélange final des poudres est comprimé sur une presse rotative équipée de poinçons de 13 mm de diamètre, pour l'obtention de comprimés répondant à la formule unitaire précitée.

**Exemple 2**

Comprimés effervescents

| | | |
|---|---:|---|
| Extrait de ginseng | 50 | mg |
| (correspondant à 250 mg de poudre) | | |
| N-acétylasparaginate d'arginine | 250 | mg |
| Bicarbonate sodique | 375 | mg |
| Acide citrique anhydre | 450 | mg |
| $\alpha$-lactose anhydre | 512,5 | mg |
| Benzoate sodique | 50 | mg |
| Saccharinate sodique | 5 | mg |
| Arôme | 7,5 | mg |

Après avoir tamisé le saccharinate sodique et l'extrait de ginseng, un prémélange est réalisé avec une partie d'α-lactose anhydre, l'arôme et le saccharinate sodique. Le prémélange est ensuite mélangé au N-acétylasparaginate d'arginine, au bicarbonate sodique, à l'acide citrique et à la quantité restante d'α-lactose anhydre. On complète en mélangeant avec le benzoate sodique tamisé. La poudre obtenue est ensuite comprimée directement.

**Exemple 3**

Solution buvable

| | | |
|---|---:|---|
| Extrait de ginseng | 100 | mg |
| (correspondant à 500 mg de poudre) | | |
| N-acétylasparaginate d'arginine | 500 | mg |
| Saccharinate sodique | 0,3 | mg |
| Arôme de fruits exotiques | 12,5 | mg |
| Nipagine® | 4 | mg |
| Nipasol® | 1 | mg |
| Propylène glycol | 45 | mg |
| Vin de quinquina qs ad | 5 | ml |

Les conservateurs Nipagine® et Nipasol® sont dissous dans le propylène glycol. On y ajoute ensuite l'arôme de fruits exotiques. Par ailleurs, une solution, dans le vin de quinquina, d'extrait de ginseng, de N-acétylasparaginate d'arginine et de saccharinate sodique est réalisée et ajoutée à la solution précédente de propylène glycol. On complète le volume final avec du vin de quinquina.

Une étude clinique randomisée comparant, dans une population de 30 patients présentant de l'asthénie, l'efficacité du ginseng, de l'acétylasparaginate d'arginine et de l'association de ginseng et d'acétylasparaginate d'arginine a été effectuée, la durée du traitement étant de deux semaines. Les paramètres d'évaluation concernaient l'asthénie et le rendement intellectuel ; ils ont été contrôlés à l'état de base et à la fin du traitement. Il ressort de cette étude que l'association de ginseng et d'acétylasparaginate d'arginine est significativement plus efficace que les deux substances de comparaison administrées en monothérapie.

Cette différence est statistiquement significative en ce qui concerne les échelles quantitatives adoptées pour l'évaluation de l'asthénie et du rendement intellectuel. Sur le plan des effets secondaires, l'étude confirme l'excellente tolérance du ginseng et de l'acétylasparaginate d'arginine administrés en association.

## Revendications

1. Composition antiasthénique, caractérisée en ce qu'elle comprend, comme ingrédient actif, un mélange de ginseng et de N-acétylasparaginate d'arginine, en association avec un excipient pharmacologiquement acceptable.

2. Composition suivant la revendication 1, caractérisée en ce que le ginseng est sous forme de poudre.

3. Composition suivant la revendication 2, caractérisée en ce que le rapport en poids de la poudre de ginseng par rapport au N-acétylasparaginate d'arginine est de l'ordre de 1/1.

4. Composition suivant la revendication 1, caractérisée en ce que le ginseng est sous forme d'extrait.

5. Composition suivant la revendication 4, caractérisée en ce que le rapport en poids de l'extrait de ginseng par rapport au N-acétylasparaginate d'arginine est de l'ordre de 1/5.

6. Composition suivant l'une quelconque des revendications 1 à 5, caractérisée en ce que l'arginine du N-acétylasparaginate d'arginine est de l'arginine lévogyre, de l'arginine dextrogyre ou un mélange de ces arginines.

7. Composition suivant l'une quelconque des revendications 1 à 3 et 6, caractérisée en ce qu'elle comprend, comme excipient, une ou plusieurs substances choisies dans le groupe comprenant la polyvinylpyrrolidone, la cellulose microcristalline, le stéarate de magnésium, le bicarbonate sodique, l'acide citrique anhydre, l'$\alpha$-lactose anhydre, le benzoate sodique et le saccharinate sodique.

8. Composition suivant la revendication 7, caractérisée en ce que l'excipient précité est un mélange de polyvinylpyrrolidone, de cellulose microcristalline et de stéarate de magnésium.

9. Composition suivant la revendication 7, caractérisée en ce que l'excipient précité est un mélange de bicarbonate sodique, d'acide citrique anhydre, d'$\alpha$-lactose anhydre, de benzoate sodique et de saccharinate sodique.

10. Composition suivant l'une quelconque des revendications 1 à 9, caractérisée en ce qu'elle se présente sous la forme d'une dose unitaire ou posologique à usage oral.

11. Composition suivant la revendication 10, caractérisée en ce qu'elle se présente sous forme de comprimés effervescents ou non ou de gélules.

12. Composition suivant la revendication 11, caractérisée en ce qu'un comprimé ou une gélule comprend environ 250 mg de poudre de ginseng et environ 250 mg de N-acétylasparaginate de L-arginine.

13. Composition suivant l'une ou l'autre des revendications 11 et 12, caractérisée en ce qu'un comprimé contient, comme excipient, environ 25 mg de polyvinylpyrrolidone, environ 25 mg de cellulose microcristalline et environ 2,5 mg de stéarate de magnésium.

14. Composition suivant l'une quelconque des revendications 1 et 4 à 6, caractérisée en ce qu'elle comprend, comme excipient, du vin de quinquina et en ce qu'elle se présente sous la forme de solution buvable.

15. Procédé de préparation de la composition antiasthénique suivant l'une quelconque des revendications 1 à 3 et 6 à 13, caractérisé en ce qu'on mélange le ginseng et le N-acétylasparaginate d'arginine avec l'excipient pharmacologiquement acceptable et en ce qu'on donne au mélange une forme appropriée à son administration par voie orale, telle que celle de comprimés ou de gélules, la dose unitaire en chacun de ces constituants actifs de ladite composition étant de l'ordre de 250 mg.

16. Utilisation de la composition suivant l'une quelconque des revendications 1 à 14, pour la préparation d'un médicament pour le traitement de l'asthénie.

## Patentansprüche

1. Anti-asthenische Zusammensetzung, dadurch gekennzeichnet, daß sie als aktiven Bestandteil eine Mischung von Ginseng und Arginin-N-adetylasparaginat zusammen mit einer pharmazeutisch annehmbaren Trägersubstanz enthält.

2. Zusammensetzung nach Anspruch 1, dadurch gekennzeichnet, daß der Ginseng in Form eines Pulvers vorliegt.

3. Zusammensetzung nach Anspruch 2, dadurch gekennzeichnet, daß das Gewichtsverhältnis des Ginseng-Pulvers zum Arginin-N-acetylasparagimat in der Größenordnung von 1/1 ist.

4. Zusammensetzung nach Anspruch 1, dadurch gekennzeichnet, daß der Ginseng in Form eines Extraktes vorliegt.

5. Zusammensetzung nach Anspruch 4, dadurch gekennzeichnet, daß das Gewichtsverhätnis des Ginseng-Extraktes zum Arginin-N-acetylasparaginat in der Größenordnung von 1/5 ist.

6. Zusammensetzung nach einem der Ansprüche 1 bis 5, dadurch gekennzeichnet, daß das Arginin des Arginin-N-acetylasparaginats linksdrehendes Arginin, rechtsdrehendes Arginin, oder eine Mischung dieser Arginine ist.

7. Zusammensetzung nach einem der Ansprüche 1 bis 3 und 6, dadurch gekennzeichnet, daß sie als Trägersubstanz eine oder mehrere Substanzen enthält, ausgewählt aus der Gruppe umfassend Polyvinylpyrrolidon, mikrokristalline Cellulose, Magnesiumstearat, Natriumbicarbonat, wasserfreie Zitronensäure, wasserfreie $\alpha$-Lactose, Natriumbenzoat und Natriumsaccharinat.

8. Zusammensetzung nach Anspruch 7, dadurch gekennzeichnet, daß die Trägersubstanz eine Mischung aus Polyvinylpyrrolidon mikrokristalliner Cellulose und Magnesiumstearat ist.

9. Zusammensetzung nach Anspruch 7, dadurch gekennzeichnet, daß die Trägersubstanz eine Mischung von Natriumbicarbonat, wasserfreier Zitronensäure, wasserfreier $\alpha$-Lactose, Natriumbenzoat und Natriumsaccharinat ist.

10. Zusammensetzung nach einem der Ansprüche 1 bis 9, dadurch gekennzeichnet, daß sie in Form einer Einheitsdosis oder Dosierung für die orale Verwendung vorliegt.

11. Zusammensetzung nach Anspruch 10, dadurch gekennzeichnet, daß sie in Form von Brausetabletten oder Nicht-Brausetabletten, oder von Kapseln vorliegt.

12. Zusammensetzung nach Anspruch 11, dadurch gekennzeichnet, daß eine Tablette oder Kapsel ca. 250 mg Gingseng-Pulver und ca. 250 mg L-Arginin-N-acetylasparaginat enthält.

13. Zusammensetzung nach einem der Ansprüche 11 und 12, dadurch gekennzeichnet, daß eine Tablette als Trägersubstanz ca. 25 mg Polyvinylpyrrolidon ca. 25 mg mikrokristalline Cellulose und ca. 2.5 mg Magnesiumstearat enthält.

14. Zusammensetzung nach einem der Ansprüche 1 und 4 bis 6, dadurch gekennzeichnet, daß sie als Trägersubstanz Chinarindenwein enthält, und dadurch, daß sie in Form einer trinkbarer Lösung vorliegt.

15. Verfahren zur Herstellung der anti-asthenischen Zusammensetzung nach einem der Ansprüche 1 bis 3 und 3 und 6 bis 13, dadurch gekennzeichnet, daß man den Ginseng und das Arginin-N-acetylasparaginat mit der pharmazeutisch annehmbaren Trägersubstanz mischt und man die Mischung in eine zur oralen Verabreichung geeignete Form überführt, wie z.B. in Tabletten oder Kapseln, wobei die Einheitsdosis von jedem dieser aktiven Bestandteile dieser Zusammensetzung in der Größenordnung von 250 mg ist.

16. Verwendung der Zusammensetzung nach einem der Ansprüche 1 bis 14 zur Herstellung eines Arzneimittels zur Behandlung von Astehnie.

## Claims

1. An antiasthenic composition, characterised by the fact that it contains, as an active ingredient, a mixture of ginseng and N-acetylasparaginate of arginine, combined with a pharmacologically acceptable excipient.

2. A composition according to claim 1, characterised by the fact that the ginseng is in the form of powder.

3. A composition according to claim 2, characterised by the fact that the ratio by weight of the ginseng powder in relation to the N-acetylasparaginate of arginine is of the order of 1 : 1.

4. A composition according to claim 1, characterised by the fact that the ginseng is in the form of an extract.

5. A composition according to claim 4, characterised by the fact that the ratio by weight of the ginseng extract in relation to the N-acetylasparaginate of arginine is of the order of 1 : 5.

6. A composition according to any of claims 1 to 5, characterised by the fact that the arginine of the N-acetylasparaginate of arginine is laevorotaty arginine, dextrorotatory arginine or a mixture of these arginines.

7. A composition according to any of claims 1 to 3, and 6, characterised by the fact that it contains, as excipient, or more substances chosen from the group including polyvinyl pyrrolidone, microcrystalline cellulose, magnesium stearate, sodium bicarbonate, anhydrous citric acid, anhydrous $\alpha$-lactose, sodium benzoate and sodium saccharinate.

8. A composition according to claim 7, characterised by the fact that the aforementioned excipient is a mixture of polyvinyl pyrrolidone, microcrystalline cellulose and magnesium stearate.

9. A composition according to claim 7, characterised by the fact that the aforementioned excipient is a mixture of sodium bicarbonate, anhydrous citric acid, anhydrous α-lactose, sodium benzoate and sodium saccharinate.

10. A composition according to any of claims 1 to 9, characterised by the fact that it comes in the form of a unitary or posoligical dose to be taken orally.

11. A composition according to claim 10, characterised by the fact it comes in the form of effervescent or non-effervescent tablets, or capsules.

12. A composition according to claim 11, characterised by the fact that one tablet or capsule contains approximately 250 mg of ginseng powder and approximately 250 mg of N-acetylasparaginate of L-arginine.

13. A composition according to either of claims 11 and 12, characterised by the fact that one tablet contains, as excipient, approximately 25 mg of polyvinyl pyrrolidone, approximately 25 mg of microcrystalline cellulose and approximately 205 mg of magnesium stearate.

14. A composition according to any of claims 1 and 4 to 6, characterised by the fact that it contains, as excipient, quinine tonic wine and that it comes in the form of a solution to be taken orally.

15. A process of preparing the antiasthenic composition according to any of claims 1 to 3 and 6 to 13, characterised by the fact that ginseng and N-acetylasparaginate of arginine are mixed with the pharmacologically acceptable excipient and that the mixture is given a suitable form for oral administration, i.e. tablets or capsules, the unitary dose of each of these active constituents of the said composition being of the order of 250 mg.

16. Use of the composition according to any of claim 1 to 14, for the preparation of a medicine for treating asthenia.